# EUROPÄISCHE PATENTSCHRIFT

(11) **EP 0 885 904 B1**
(45) Veröffentlichungstag und Bekanntmachung des Hinweises auf die Patenterteilung: **24.03.2004**
(21) Anmeldenummer: 98110629.7
(22) Anmeldetag: 10.06.1998
(51) Int. Cl.: C07K 14/575, C07K 7/06, C07K 14/47, A61K 38/22

(54) **Peptide als Agonisten und/oder Inhibitoren der Amyloidbildung und/oder Zytotoxizität sowie der Verwendung bei der Alzheimer'schen Krankheit, beim Typ II Diabetes mellitus und bei spongiformen Encephalopathien**
Peptides as agonists and/or inhibitors of amyloid formation and/or cytotoxicity and their use against Alzheimer's disease, type II diabetes mellitus and spongiform encephalopathy
Peptides agonistes et/ou inhibiteurs de la formation d'amyloide et/ou de cytotoxicité et leur utilisation contre la maladie d'Alzheimer, le diabète sucré de type II et l'encéphalopathie spongiforme

(30) Priorität: 17.06.1997 DE 19725619
(43) Veröffentlichungstag der Anmeldung: 23.12.1998
(73) Patentinhaber: Fraunhofer-Gesellschaft zur Förderung der angewandten Forschung e.V., 80686 München (DE)
(72) Erfinder: Kapurnioutu, Afroditi, 72074 Tübingen (DE); Bernhagen, Jürgen, 72074 Tübingen (DE); Brunner, Herwig, 70569 Stuttgart (DE)

(56) Entgegenhaltungen:
- EP-A- 0 289 287
- EP-A- 0 309 100
- WO-A-96/07425
- WO-A-96/39834
- K. GANOWIAK ET AL: "Fibrils from synthetic amyloid-related peptides enhance development of experimental AA-amyloidosis in mice" BIOCHEMICAL AND BIOPHYSICAL RESEARCH COMMUNICATIONS., Bd. 199, Nr. 1, 1994, Seiten 306-312, XP002077761 ORLANDO, FL US
- HUGHES ET AL: 'Inhibition of toxicity in the beta-amyloid Peptide Fragment beta-(25-35) Using N-Methylated Derivatives' THE JOURNAL OF BIOLOGICAL CHEMISTRY Bd. 275, Nr. 33, 18 August 2000, Seiten 25109 - 25115

## Beschreibung

Die Erfindung betrifft Peptide mit bis zu 15 Aminosäuren, die als Agonisten und/oder Inhibitoren der Amyloidbildung und/oder Toxizität fungieren und deshalb bei diesbezüglich verschiedenen Krankheitsbildern eingesetzt werden können.

Neue Strategien und Wirkstoffe zur Therapie und Diagnose der o.g. amyloiden Krankheiten sind weltweit stark umforscht. Eine Möglichkeit zur Behandlung dieser Krankheiten mit Medikamenten/Pharmazeutika gibt es jedoch noch nicht. Der vorherrschenden Lehrmeinung nach sind bestimmte, für jede Krankheit spezifische amyloide Proteine durch ihre Amyloidogenese oder Aggregation ursächlich verantwortlich für das Entstehen der amyloiden Krankheiten. Der Mechanismus zur Amyloidogenese und dem damit zusammenhängenden Zelltod (Zytotoxizität) bei diesen Krankheiten ist jedoch weitgehend unbekannt und entsprechende hochspezifische Inhibitoren sind daher noch nicht identifiziert worden. Pharmazeutika zur Behandlung der amyloiden Krankheiten auf der Basis solcher Inhibitoren wurden also ebenfalls noch nicht entwickelt.

Ebenso haben die durch die Amyloidbildung (Bildung von unlöslichen Proteinaggregaten, den sogenannten amyloiden Strukturen) verursachten proteinchemischen, technisch-analytischen Probleme bisher keine Analytik der Amyloidbildung erlaubt. Das hat dazu beigetragen, dass der Mechanismus der Amyloidbildung noch weitgehend unaufgeklärt ist. Auf der Amyloidbildungsanalyse aufbauende diagnostische Verfahren (schnelle in vitro-Tests zur Bewertung von Menge, Zeitablauf und Qualität der amyloiden Strukturen) existieren daher bei den genannten Krankheiten ebenfalls noch nicht. Zum Beispiel kann eine Diagnose bei der Alzheimerschen nur symptomatisch (ansteigende Vergesslichkeit oder ähnliches) oder post mortem durchgeführt werden. es können z.B. keine zuverlässigen Bluttests zu Lebzeiten der Patienten durchgeführt werden.

Ausgehend hiervon ist es deshalb die Aufgabe der Erfindung, geeignete Peptide vorzuschlagen, die als Agonisten und/oder Inhibitoren der Amyloidbildung und/oder Zytotoxizität fungieren können.

Die Aufgabe wird durch die Peptide gemäß Anspruch 1, 2 oder 3 gelöst. Die Unteransprüche zeigen vorteilhafte Weiterbildungen auf. Die Verwendung der Peptide ist in den Ansprüchen 7 bis 10 angegeben.

Es hat sich gezeigt, dass die erfindungsgemäßen Peptidmoleküle als Inhibitoren derjenigen amyloiden Peptide/Proteine fungieren, welche die amyloiden Krankheiten Alzheimer'sche Krankheit, Typ II Diabetes mellitus und Spongiforme Encephalopathien (z.B. Creutzfeld-Jacob-Krankheit, Scrapie Krankheit, BSE) verursachen. Die erfindungsgemäßen Peptidmoleküle sind in der Lage, die Amyloidogenese oder Aggregation der amyloiden Peptide/Proteine β-amyloides Peptid oder β-AP (bei der Alzheimer'schen Krankheit), Amylin/IAPP (beim Typ II Diabetes mellitus) und Prion-Protein (bei den spongiformen Encephalopathien) zu hemmen. Durch die erreichte Inhibition der Amyloidogenese der krankheitsverursachenden Peptide/Proteine wird auch die durch aggregiertes β-AP, Amylin/IAPP oder Prion-Protein verursachte zytotoxische Wirkung auf Gewebszellen inhibiert.

Die Erfindung bietet folgende Vorteile gegenüber dem Stand der Technik:
- Einfache Chemosynthese in hoher Reinheit und Ausbeute nach gängigen Methoden der Festphasenpeptidsynthese (die hier beschriebenen Peptide sind i.d.R. kürzer als die bisher beschriebenen potentiellen Inhibitoren und bestehen aus nur einer Art chemischer Bausteine [= Aminosäuren].
- Hohe biologische Stabilität, die durch den einfachen chemischen Einbau unnatürlicher Aminosäuren noch erhöht werden kann.
- Breite biologische und damit potentiell therapeutische Anwendbarkeit (der hohe Homologiegrad zwischen den entsprechenden Sequenzen der amyloidbildenden Peptide/Proteine ermöglicht den überlappenden Einsatz der Inhibitoren in allen drei Krankheiten.
- Geringe Nebenwirkungen und geringe Antigenizität beim Einsatz als Therapeutikum (die Peptide weisen aufgrund ihrer geringen Größe eine kleine Tendenz zur Induktion von Immunreaktionen im Patienten auf; andere in der Literatur beschriebene Inhibitorkandidaten (siehe oben Antikörper oder höhermolekulare Serumkomponenten und sind ca. 200-300 mal größer als die hier beschriebenen Peptide).
- Hohe biologische Aktivität in vitro und damit hohe vorhersagbare biologische Aktivität in vivo.
- Damit hohe Vorhersagbarkeit einer therapeutischen Anwendung.

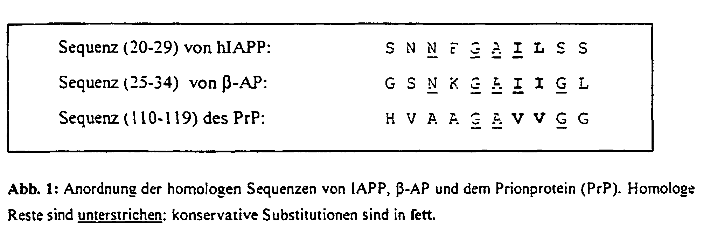

Es hat sich gezeigt, dass für die drei eingangs beschriebenen Anwendungsgebiete jeweils unterschiedliche Peptide besonders geeignet sind, die jedoch alle untereinander Homologie aufweisen (Abb. 1). Nachfolgend werden die einzelnen Peptide für die drei Gruppen näher erläutert.

Eine geeignete Peptidsequenz, welche für die Hemmung der Amyloidbildung und Zytotoxizität von IAPP ausreichend ist, ist die Sequenz FGAIL (Einbuchstabencode), welche die Aminosäurereste 23 - 27 von IAPP umfasst. Eine Verlängerung dieser Sequenz in Richtung des N-Terminus von IAPP ergibt gleichfalls kleine (<10 Aminosäurereste) Peptidfragmente, welche Fibrillen (eine geordnete Aggregatstruktur, die typisch für amyloide Krankheiten ist) bilden können und Zytotoxizität aufweisen. Zur spontanen Aggregation, d.h. Herstellung einer übergesättigten Peptidlösung dieser Peptide, wird jedoch eine 100fach höhere Konzentration, verglichen mit IAPP, benötigt. Diese Sequenzen wurden als kleinmolekulare Inhibitoren der Amyloidbildung von IAPP erfolgreich eingesetzt, da sie die kürzeste und für die Aggregation von IAPP notwendige Peptidsequenz enthalten. Diesem Effekt liegt der Aggregationsmechanismus von IAPP und gleichermaßen anderer amyloidbildender Peptide (wie β-AP und dem Prion-Protein) zugrunde. Dabei erfolgt die Aggregation und Amyloidbildung durch eine intermolekulare β-Faltblatt-Bildung, wofür intermolekulare (zwischen den Molekülketten) Wasserstoffbrücken und hydrophobe Wechselwirkungen zwischen den Seitenketten bestimmter Aminosäurereste notwendig sind. Diese β-Faltblatt-Struktur führt zu einer nicht-konvalenten Bindung zwischen zunächst zwei und anschließend mehreren IAPP-Molekülen, welche daraufhin unlösliche Aggregate/Amyloid-Strukturen bilden. Der Wirkungsmechanismus (Inhibition) der erfindungsgemäßen Peptide besteht darin, die aggregationsfördernden, intermolekularen Wechselwirkungen zwischen zwei IAPP-Molekülen zu unterbinden. Letzteres wird dadurch erreicht, dass die Peptide selber diese Wechselwirkungen mit IAPP eingehen. Somit entsteht eine Kompetition zwischen IAPP und den Peptiden um die freien "Bindungsstellen" von IAPP. Auf dieser Wirkung dieser Peptide beruht auch ihr potentieller Einsatz als Krankheits-Diagnostika. Dadurch, dass die lösliche Form der Peptide und bzw. niedrige Konzentrationen (mikromolar) weder aggregieren noch Zytotoxizität aufweisen, wird ihr Einsatz als Inhibitoren und Typ II-Diabetes-Diagnostika ermöglicht.

Folgende Peptidsequenzen wurden synthetisiert und als Inhibitoren der Amyloidbildung und Zytotoxizität von IAPP eingesetzt: FGAIL, NFGAIL, NNFGAIL, SNNFGAIL, NFGAILSS, SNNFGAILSS, wobei die einzelnen Buchstaben für Aminosäuren nach dem Einbuchstabencode stehen. Darüber hinaus werden H-Moleküle der Amidbindungen der obigen Peptidsequenzen durch eine Methylgruppe ersetzt, um die durch β-Faltblattbildung induzierte Aggregation der Peptide zu unterbinden. Die MethylGruppen wurden bei jeder zweiten Amidbindung eingeführt, jedoch variierte die Zahl der eingeführten Methyl-Gruppierungen. Die hergestellten Peptidanaloga enthalten eine Zahl von N-methylsubstituierten Amidbindungen, welche sich zwischen einer und der Hälfte der vorhandenen Amidbindungen pro Molekül erstreckt. Dem Design dieser Klasse von Aggregationsinhibitoren liegt die Überlegung zugrunde, dass durch die Einführung der Methylgruppe bei jeder zweiten Amidbindung die Dimerisierung oder die nicht-kovalente Bindung der kleinmolekularen Peptidsequenzen an IAPP nicht beeinträchtigt, die sonst nachfolgende nicht-kovalente Expandierung der β-Faltblattstrukturen, welche zur Aggregation führt, jedoch weitgehend ausgeschaltet wird. Im folgenden werden repräsentative Beispiele dieser Substanzklasse, welche als Aggregationsinhibitoren von IAPP erfolgreich eingesetzt wurden, benannt: F(N-Me)-GA-(N-Me)IL, NF(N-Me)-GA-(N-Me)IL, NNF(N-Me)-GA-(N-Me)IL, SNNF (N-Me)-GA-(N-Me) IL, NF(N-Me)-GA-(N-Me) ILSS, SNNF(N-Me)-GA-(N-Me) IL, FG(N-Me)-AI(N-Me)-L, NFG(N-Me)-AI(N-Me)-L, NNFG(N-Me)-AI(N-Me)-L, SNNFG(N-Me)-AI(N-Me)-L, NFG (N-Me)AI(N-Me)-LSS, SNNFG(N-Me)-AI(N-Me)-LSS, FGA(N-Me)-IL, NFGA(N-Me)-IL, NNFGA(N-Me)-IL, SNNFGA(N-Me)-IL, NFGA(N-Me)-ILSS, SN(N-Me)-NFGAILSS, (N-Me)-SN(N-Me)NFGAILSS, (N-Me)-SN(N-Me)-NF(N-Me)-GAILSS. Bei einem geeigneten Peptid, welches sich für die Amyloidbildung und Zytotoxizität von β-AP einsetzen lässt, handelt es sich um Sequenzbereiche von β-AP, welche sich zwischen den Aminosäuren 25 und 34 befinden. In Analogie zur Sequenz 23 - 27 von IAPP wurde in β-AP die Sequenz 28 - 32 (KGAII) als eine geeignete Sequenz, welche für die Amyloidbildung und Neurotoxizität des Gesamtmoleküls β-AP ausreicht, gefunden. Aus den gleichen Überlegungen wie im Falle der IAPP-Amyloidbildungsinhibitoren wurden die folgenden zum IAPP homologen β-AP Sequenzen synthetisiert und als Inhibitoren der β-AP-Amyloidbildung und - Neurotoxizität eingesetzt: KGAII, NKGAII, SNKGAII, GSNKGAII, NKGAIIGL, GSNKGAIIGL. Fernerhin, wie oben beschrieben, wurden auch Analoga mit substituierten Amidbindungen hergestellt. Die Strukturen dieser Analoga wurden nach dem gleichen Prinzip wie die N-methylierten Inhibitoren der IAPP-Amyloidbildung (siehe oben) entworfen. Im folgenden werden repräsentative Beispiele dieser Substanzklasse aufgelistet: K(N-Me)-GA-(N-Me)II, NK(N-Me)-GA-(N-Me)-II, SNK(N-Me)-GA-(N-Me) II, GSNK(N-Me)-GA-(N-Me)II, NK(N-Me)-GA-(N-Me)IIGL, GSNK(N-Me)-GA-(N-Me)II, KG(N-Me)-AI(N-Me)-I, NKG(N-Me)-AI(N-Me)-I, SNKG(N-Me)-AI(N-Me)-I, GSNKG(N-Me)-AI(N-Me)-I, NKG(N-Me)-AI(N-Me)-IGL, GSNKG(N-Me)-AI(N-Me)-IGL, KGA(N-Me)-II, NKGA(N-Me)-II, SNKGA(N-Me)-II, GSNKG-A(N-Me)-IIGL, GS(N-Me)-NKGAIIGL, (N-Me)-GS(N-Me)-NKGAIIGL, (N-Me)-GS(N-Me)-NK(N-Me)-GAIIGL.

Eine geeignete Sequenz, welche für die Amyloidbildung und Zytotoxizität von PrP ausreichend ist, ist AGAVV. Es handelt sich hier um eine Teilsequenz aus der Sequenz 110 - 119 PrP. Weitere Sequenzen, die als Aggregations- und Toxizitätsinhibitoren von PrP Verwendung finden, sind: AAGAVV, VAAGAVV, HVAAGAVV, AAGAVVGG, HVAAGAVVGG. Auch die entsprechenden N-methylierten Peptidsequenzen - analog zu den IAPP-Derivaten (siehe oben) sind als Aggregations- und Toxizitätsinhibitoren geeignet. Im folgenden werden einige repräsentative Beispiele der N-methylierten Analoga aufgeführt. A(N-Me)-GA-(N-Me)VV, AA(N-Me)-GA-(N-Me)VV, AAGA(N-Me)-VVGG, HV(N-Me)-AAGAVVGG, (N-Me)-KV(N-Me)-AAGAVVGG, (N-Me)-HV-(N-Me)-AA(N-Me)-GAVVGG etc.

Bei der Erfindung handelt es sich somit um peptidische Moleküle, welche als Grundstrukturen (Templatmoleküle) zur Inhibition und Analyse der Amyloidbildung und Inhibition der Zytotoxizität bei amyloiden Krankheiten verwendet werden können. Dabei wirken diese Peptide auf die für die amyloiden Krankheiten verantwortlichen Moleküle (ihrerseits amyloidbildende Peptide und Proteine) ein. Die Peptide sind somit entweder selber Inhibitoren der Amyloidbildung und Zytotoxizität oder können als Template für die Identifizierung und Herstellung weiterer Inhibitoren und Agonisten dienen oder können als molekulare Werkzeuge bei der Analyse eingesetzt werden.

Diese Peptide finden Anwendung als pharmazeutische Inhibitoren der Amyloidbildung und Zytotoxizität oder als molekulare Werkzeuge zur Analyse der Amyloidbildung und Zytotoxizität bei amyloiden Krankheiten. Somit handelt es sich um potentielle Pharmaka und Analytika zur Behandlung und Diagnose folgender auch beim Menschen vorkommender Krankheiten:
Alzheimer'sche Krankheit,
Typ II Diabetes mellitus,
Spongiforme Encephalopathien
(z.B. Creutzfeld-Jacob-Krankheit, Scrapie-Krankheit, BSE).

Die diagnostische Anwendung umfaßt zwei Aspekte:
- Verwendung als molekulares Werkzeug zur weiteren Erforschung des Mechanismus der Amyloidbildung bei diesen Krankheiten (Einsatz in Forschungslabors und F&E-Labors).
- Potentielle Verwendung als Reagenz zur Diagnostik amyloider Krankheiten bzw. der Vorhersage solcher Krankheiten (Diagnostika-Markt).
   Die Erfindung wird nachfolgend anhand eines Ausführungsbeispiels näher erläutert.
   NFGAIL: Herstellung, Charakterisierung, Aggregation, Testung der Fribrillenbildung, Testung der Zytotoxizität, Testung der inhibitorischen Wirkung auf die Aggregation von IAPP.

### - Herstellung und Charakterisierung

NFGAIL wurde nach gängigen Methoden der Festphasenpeptidsynthese hergestellt. Es wurde der Wang-Anker verwendet, und die Fmoc/tBu-Synthesestrategie eingesetzt. Für einen 1 mM Ansatz wurden 4 mmol der geschützten Aminosäuren 4 mmol TBTU und 6 mmol DIEA in DMF pro Kupplungsschritt verwendet. Die Abspaltung der temporären Schutzgruppen (Fmoc) erfolgte mittels 25 % Piperidin in DMF und für die Abspaltung des Peptides vom Anker unter gleichzeitiger Abspaltung der permanenten Schutzgruppen der Seitenketten wurde 95 % TFA verwendet (Reaktionszeit 2 h). Das Rohrprodukt wurde durch präparative RP-HPLC gereinigt und durch FAB-MS und Aminosäureanalyse charakterisiert.

### - Aqqregration und Testung der Fibrillenbildung

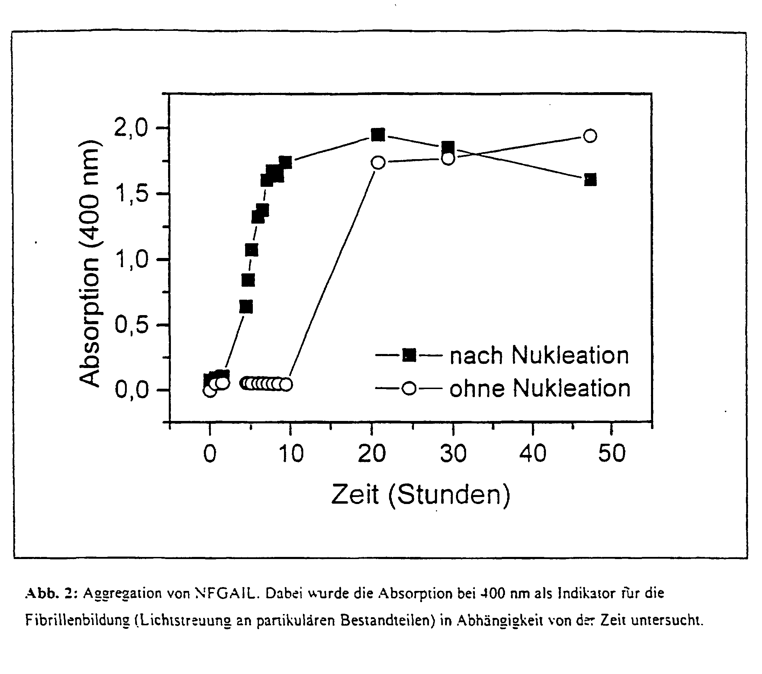

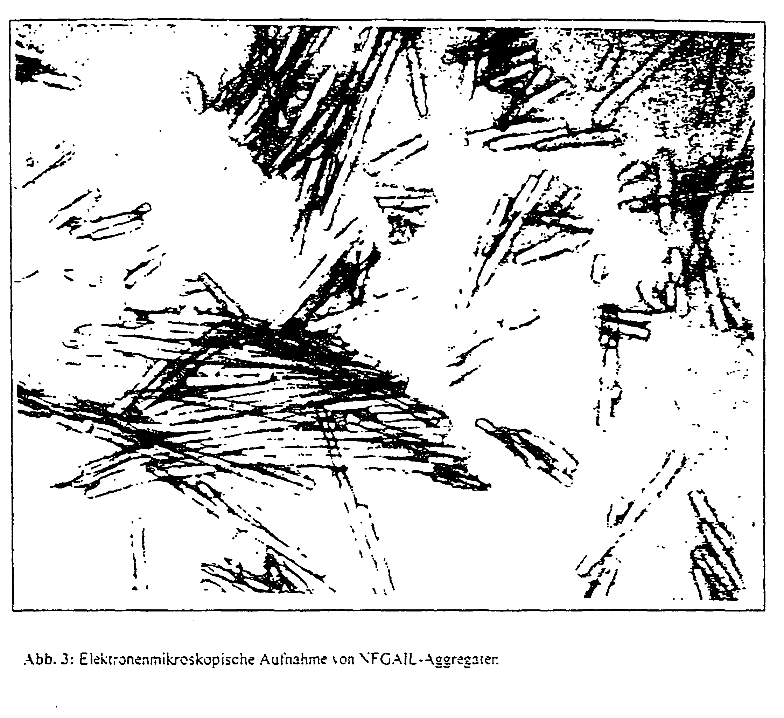

Die Aggregationseigenschaften wurden in 10 mM Phosphatpuffer pH 7.4 getestet. Zunächst wurde eine hochkonzentrierte (125 - 250 mM) Stammlösung des Peptides in DMSO hergestellt. Aus dieser Lösung wurde das Peptid direkt in die Aggregationslösung unter mildem Rühren pipettiert. Eine 5 mM Peptidlösung aggregierte spontan und wies somit keine "Aggregations-Lag-Time" auf. Dagegen hat sich ein Aggregat erst nach 9 1/2 h gebildet aus einer Lösung von 3,75 mM NFGAIL (Aggregations-Lag-Time: 9 1/2 h) (Abb. 2). Fernerhin erfolgte sofortige Aggregation, wenn vorgefertigte NFGAIL.,Fibrillen (Nukleationskeime) zu der 3,75 mM Lösung addiert wurden (Fibrillenkonzentration 0,375 mM). Somit wurde gezeigt, daß die Aggregation dieser Peptide nach dem sogenannten "nukleationsabhängigen Polymerisationsmechanismus" verläuft. Letzteres ist von großer Bedeutung, da in der Literatur die Meinung herrscht, daß die Amyloidbildung (in vitro) durch diesen Mechanismus verlaufen soll. Nach vollständiger Aggregation wurde das Präzipitat durch Zentrifugation isoliert und durch Elektronen- und Polarisationsmikrokospie nach Kongorot Färbung untersucht. Dadurch wurde die fibrilläre und amyloide Struktur des Aggregats bestätigt (Abb. 3).

### - Testung der Zytotoxizität

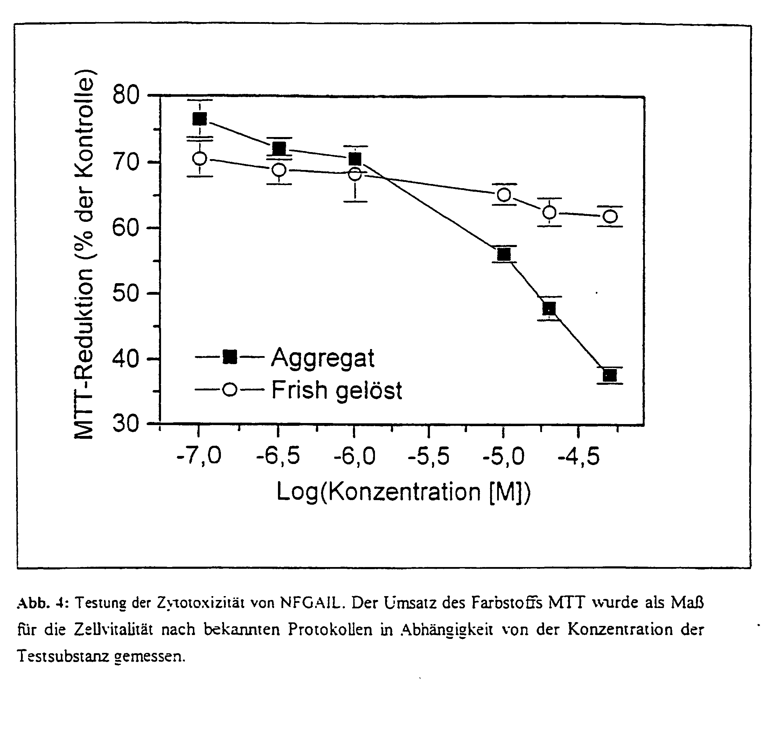

Suspensionen der Peptidaggregate (hergestellt wie oben beschrieben) wurden mittels einer Rat Insulinoma (RIN5mf) und einer humanen Astroglioma-Zellinie (HTB-14) auf Toxizität getestet. Die frisch gelösten Peptide wurden ebenso auf Zytotoxizität getestet. Somit konnte gezeigt werden, daß die aggregierte Form von NFGAIL zytotoxisch ist, wobei die lösliche Form des Peptides keine Toxizität aufwies (Abb. 4).

## Patentansprüche

1. Peptid mit bis zu 15 Aminosäuren, **dadurch gekennzeichnet, dass** es eine der folgenden Peptidsequenzen VAAGAVV, HVAAGAW oder HVAAGAWGG enthält.

2. Peptid, **dadurch gekennzeichnet, dass** es durch eine der folgenden Peptidsequenzen
FGAIL, SNNFGAIL, NFGAILSS, KGAII, NKGAII, SNKGAII, GSNKGAII, NKGAIIGL, GSNKGAIIGL, AGAVV, AAGAVV, VAAGAVV, HVAAGAVV, AAGAVVGG oder HVAAGAVVGG
gebildet ist.

3. Peptid mit bis zu 15 Aminosäuren, **dadurch gekennzeichnet, dass** es eine der folgenden Peptidsequenzen
FGAIL, NFGAIL, NNFGAIL, SNNFGAIL, NFGAILSS, SNNFGAILSS, KGAII, NKGAII, SNKGAII, GSNKGAII, NKGAIIGL, GSNKGAIIGL, AGAW, AAGAVV, VAAGAVV, HVAAGAVV, AAGAVVGG oder HVAAGAVVGG enthält und bei mindestens einer Amidbindung das Wasserstoffmolekül durch eine Methylgruppe ersetzt ist.

4. Peptide nach Anspruch 3, **dadurch gekennzeichnet, dass** es durch eine der folgenden Peptidsequenzen
FGAIL, NFGAIL, NNFGAIL, SNNFGAIL, NFGAILSS, SNNFGAILSS, KGAII, NKGAII, SNKGAII, GSNKGAII, NKGAIIGL, GSNKGAIIGL, AGAW, AAGAVV, VAAGAVV, HVAAGAW, AAGAWGG oder HVAAGAVVGG gebildet ist und
bei mindestens einer Amidbindung das Wasserstoffmolekül durch eine Methylgruppe ersetzt ist.

5. Peptid nach Anspruch 3 oder 4, **dadurch gekennzeichnet, dass** bei jeder zweiten Amidbindung das Wasserstoffmolekül durch eine Methylgruppe ersetzt ist, jedoch mit einer Zahl von N-methylsubstituierten Amidbindungen, welche zwischen einer und der Hälfte der Amidbindungen pro Molekül beträgt.

6. Peptid nach Anspruch 3 mit einer der folgenden Sequenzen
F-(N-Me)GA-(N-Me)IL, NF-(N-Me)GA-(N-Me)IL, NNF-(N-Me)GA-(N-Me)IL, SNNF-(N-Me)GA-(N-Me)IL, NF-(N-Me)GA-(N-Me)ILSS, SNNF-(N-Me)GA-(N-Me)IL, FG-(N-Me)AI-(N-Me)L, NFG-(N-Me)AI-(N-Me)L, NNFG-(N-Me)AI-(N-Me)L, SNNFG-(N-Me)AI-(N-Me)L, NFG-(N-Me)AI-(N-Me)LSS, SNNFG-(N-Me)AI-(N-Me)LSS, FGA-(N-Me)IL, NFGA-(N-Me)IL, NNFGA-(N-Me)IL, SNNFGA-(N-Me)IL, NFGA-(N-Me)ILSS, SN-(N-Me)NFGAILSS, (N-Me)SN-(N-Me)NFGAILSS, (N-Me)SN-(N-Me)NF-(N-Me)GAILSS, K-(N-Me)GA-(N-Me)II, NK-(N-Me)GA-(N-Me)II, SNK-(N-Me)GA-(N-Me)II, GSNK-(N-Me)GA-(N-Me)II, NK-(N-Me)GA-(N-Me)IIGL, GSNK-(N-Me)GA-(N-Me)II, KG-(N-Me)AI-(N-Me)I, NKG-(N-Me)AI-(N-Me)I, SNKG-(N-Me)AI-(N-Me)I, GSNKG-(N-Me)AI-(N-Me)I, NKG-(N-Me)AI-(N-Me)IGL, GSNKG-(N-Me)AI-(N-Me)IGL, KGA-(N-Me)II, NKGA-(N-Me)II, SNKGA-(N-Me)II, GSNKGA-(N-Me)IIGL, GS-(N-Me)NKGAIIGL, (N-Me)GS-(N-Me)NKGAIIGL, (N-Me)GS-(N-Me)NK-(N-Me) GAIIGL, A-(N-Me)GA-(N-Me)VV, AA-(N-Me)GA-(N-Me)W, AAGA- (N-Me) WGG, HV- (N-Me) AAGAWGG, (N-Me)HV-(N-Me)AAGAVVGG, (N-Me)HV-(N-Me)AA-(N-Me)GAVVGG.

7. Verwendung eines Peptids mit bis zu 15 Aminosäuren, das durch eine der folgenden Peptidsequenzen
FGAIL, NFGAIL, NNFGAIL, SNNFGAIL, NFGAILSS oder SNNFGAILSS
gebildet ist oder diese enthält, zur Herstellung eines Arzneimittels als Inhibitor der Amyloidbildung und/oder der Zytotoxizität des Proteins Amylin (IAPP), zur Behandlung und/oder zur Diagnose von Typ-II-Diabetes mellitus.

8. Verwendung eines Peptids mit bis zu 15 Aminosäuren, das durch eine der folgenden Peptidsequenzen KGAII, NKGAII, SNKGAII, GSNKGAII, NKGAIIGL oder GSNKGAIIGL
gebildet ist oder diese enthält, zur Herstellung eines Arzneimittels als Inhibitor der Amyloidbildung und/oder der Zytotoxizität des Proteins β-amyloides Peptid (β-AP), zur Behandlung und/oder zur Diagnose der Alzheimer'schen Krankheit.

9. Verwendung eines Peptids mit bis zu 15 Aminosäuren, das durch eine der folgenden Peptidsequenzen
AGAVV, AAGAVV, VAAGAW, HVAAGAW, AAGAVVGG oder HVAAGAVVGG
gebildet ist oder diese enthält, zur Herstellung eines Arzneimittels als Inhibitor der Amyloidbildung und/oder der Zytotoxizität des Prion-Proteins (PrP), zur Behandlung und/oder zur Diagnose der spongiformen Enzephalopathie.

10. Verwendung eines Peptids nach einem der Ansprüche 3 bis 6
soweit diese die Sequenz GAIL enthalten zur Herstellung eines Arzneimittels als Inhibitor der Amyloidbildung und/oder der Zytotoxizität des Proteins Amylin (IAPP), zur Behandlung und/oder zur Diagnose von Typ-II-Diabetes mellitus,
soweit diese die Sequenz GAII enthalten zur Herstellung eines Arzneimittels als Inhibitor der Amyloidbildung und/oder der Zytotoxizität des Proteins β-amyloides Peptid (β-AP), zur Behandlung und/oder zur Diagnose der Alzheimer' schen Krankheit, und
soweit diese die Sequenz GAVV enthalten zur Herstellung eines Arzneimittels als Inhibitor der Amyloidbildung und/oder der Zytotoxizität des Prion-Proteins (PrP), zur Behandlung und/oder zur Diagnose der spongiformen Enzephalopathie.

## Claims

1. A peptide with up to 15 amino acids, **characterised in that** it contains one of the following peptide sequences VAAGAVV, HVAAGAVV or HVAAGAWGG.

2. A peptide, **characterised in that** it is formed from one of the following peptide sequences
FGAIL, SNNFGAIL, NFGAILSS, KGAII, NKGAII, SNKGAII, GSNKGAII, NKGAIIGL, GSNKGAIIGL, AGAVV, AAGAVV, VAAGAVV, HVAAGAVV, AAGAVVGG or HVAAGAVVGG.

3. A peptide with up to 15 amino acids, **characterised in that** it contains one of the following peptide sequences
FGAIL, NFGAIL, NNFGAIL, SNNFGAIL, NFGAILSS, SNNFGAILSS, KGAII, NKGAII, SNKGAII, GSNKGAII, NKGAIIGL, GSNKGAIIGL, AGAVV, AAGAVV, VAAGAVV, HVAAGAVV, AAGAVVGG or HVAAGAVVGG and the hydrogen atom of at least one amide bond is replaced by a methyl group.

4. A peptide according to Claim 3, **characterised in that** it is formed from one of the following peptide sequences
FGAIL, NFGAIL, NNFGAIL, SNNFGAIL, NFGAILSS, SNNFGAILSS, KGAII, NKGAII, SNKGAII, GSNKGAII, NKGAIIGL, GSNKGAIIGL, AGAVV, AAGAVV, VAAGAVV, HVAAGAVV, AAGAVVGG or HVAAGAVVGG and the hydrogen atom of at least one amide bond is replaced by a methyl group.

5. A peptide according to Claim 3 or 4, **characterised in that** the hydrogen atom of each alternate amide bond is replaced by a methyl group, but with the N-methyl substituted amide bonds amounting to a multiple of between 0.5 and 1 of the number of amide bonds per molecule.

6. A peptide according to Claim 3, having one of the following sequences
F- (N-Me) GA- (N-Me) IL, NF- (N-Me) GA- (N-Me) IL, NNF- (N-Me) GA- (N-Me) IL, SNNF- (N-Me) GA- (N-Me) IL, NF- (N-Me) GA- (N-Me) ILLS, SNNF- (N-Me) GA- (N-Me) IL, FG- (N-Me) AI - (N-Me) L, NFG- (N-Me) AI - N-Me) L, NNFG-(N-Me) AI - (N-Me) L, SNNFG- (N-Me) AI -(N-Me) L, NFG- (N-Me) AI- (N-Me) LSS, SNNFG- (N-Me) AI- (N-Me) LSS, FGA- (N-Me) IL, NFGA- (N-Me) IL, NNFGA- (N-Me) IL, SNNFGA- (N-Me) IL, NFGA- (N-Me) ILSS, SN- (N-Me) NFGAILSS, (N-Me) SN- (N-Me) NFGAILSS, (N-Me) SN- (N-Me) NF- (N-Me) GAILSS, K-(N-Me) GA- (N-Me) II, NK-(N-Me) GA- (N-Me) II, SNK-(N-Me) GA-(N-Me) II, GSNK-(N-Me) GA- (N-Me) II, NK- (N-Me) GA- (N-Me) IIGL, GSNK-(N-Me) GA- (N-Me) II, KG- (N-Me) AI (N-Me) I, NKG- (N-Me) AI (N-Me) I, SNKG- (N-Me) AI - (N-Me) I, GSNKG- (N-Me) AI - (N-Me) I, NKG- (N-Me) AI-(N-Me) IGL, GSNKG- (N-Me) AI- (N-Me) IGL, KGA- (N-Me) II, NKGA- (N-Me) II, SNKGA- (N-Me) II, GSNKGA- (N-Me) IIGL, GS- (N-Me) NKGAIIGL, (N-Me) GS- (N-Me) NKGAIIGL, (N-Me) GS- (N-Me) NK- (N-Me) GAIIGL, A- (N-Me) GA- (N-Me) VV, AA- (N-Me) GA- (N-Me) VV, AAGA- (N-Me) VVGG, HV- (N-Me) AAGAVVGG, (N-Me) HV- (N-Me) AAGAVVGG, (N-Me) HV- (N-Me) AA-(N-Me) GAVVGG.

7. Use of a peptide with up to 15 amino acids formed from or containing one of the following peptide sequences
FGAIL, NFGAIL, NNFGAIL, SNNFGAIL, NFGAILSS or SNNFGAILSS in the preparation of a medicament as an inhibitor of amyloid formation and/or of the cytotoxicity of the protein amylin (IAPP) for treating and/or diagnosing type-II diabetes mellitus.

8. Use of a peptide with up to 15 amino acids formed from or containing one of the following peptide sequences
KGAII, NKGAII, SNKGAII, GSNKGAII, NKGAIIGL or GSNKGAIIGL in the preparation of a medicament as an inhibitor of amyloid formation and/or of the cytotoxicity of the protein β-amyloid peptide (β-AP) for treating and/or diagnosing Alzheimer's disease.

9. Use of a peptide with up to 15 amino acids formed from or containing one of the following peptide sequences
AGAVV, AAGAVV, VAAGAVV, HVAAGAVV, AAGAVVGG or HVAAGAVVGG
in the preparation of a medicament as an inhibitor of amyloid formation and/or of the cytotoxicity of the prion protein (PrP) for treating and/or diagnosing spongiform encephalopathy.

10. Use of a peptide according to one of Claims 3 to 6,
insofar as the latter include the sequence GAIL for preparation of a medicament as an inhibitor of amyloid formation and/or of the cytotoxicity of the protein amylin (IAPP) for treating and/or diagnosing type-II diabetes mellitus,
insofar as the latter include the sequence GAII for preparation of a medicament as an inhibitor of amyloid formation and/or of the cytotoxicity of the protein β-amyloid peptide (β-AP) for treating and/or diagnosing Alzheimer's disease,
and
insofar as the latter include the sequence GAVV for preparation of a medicament as an inhibitor of amyloid formation and/or of the cytotoxicity of the prion protein (PrP) for treating and/or diagnosing spongiform encephalopathy.

## Revendications

1. Peptide comportant jusqu'à 15 aminoacides,
**caractérisé en ce qu'**
il renferme une des séquences peptidiques suivantes : VAAGAVV, HVAAGAVV ou HVAAGAVVGG.

2. Peptide
**caractérisé en ce qu'**
il est formé d'une des séquences suivantes :
FGAIL, SNNFGAIL, NFGAILSS, KGAII, NKGAII, SNKGAII, GSNKGAII, NKGAIIGL, GSNKGAIIGL, AGAVV, AAGAVV, VAAGAVV, HVAAGAVV, AAGAVVGG ou HVAAGAVVGG.

3. Peptide comportant jusqu'à 15 aminoacides,
**caractérisé en ce qu'**
il renferme une des séquences peptidiques suivantes :
FGAIL, NFGAIL, NNFGAIL, SNNFGAIL, NFGAILSS, SNNFGAILSS, KGAII, NKGAII, SNKGAII, GSNKGAII, NKGAIIGL, GSNKGAIIGL, AGAVV, AAGAVV, VAAGAVV, HVAAGAVV, AAGAVVGG, ou HVAAGAVVGG, et dans au moins une liaison amide, l'atome d'hydrogène est remplacé par un groupe méthyle.

4. Peptide selon la revendication 3,
**caractérisé en ce qu'**
il est formé d'une des séquences peptidiques suivantes :
FGAIL, NFGAIL, NNFGAIL, SNNFGAIL, NFGAILSS, SNNFGAILSS, KGAII, NKGAII, SNKGAII, GSNKGAII, NKGAIIGL, GSNKGAIIGL, AGAVV, AAGAVV, VAAGAVV, HVAAGAVV, AAGAVVGG, ou HVAAGAVVGG, et dans au moins une liaison amide l'atome d'hydrogène est remplacé par un groupe méthyle.

5. Peptide selon l'une quelconque des revendications 3 ou 4,
**caractérisé en ce que**
pour chaque deuxième liaison amide, l'atome d'hydrogène est remplacé par un groupe méthyle comportant cependant un nombre de liaisons amide substitué par un groupe méthyle qui s'élève entre un et la moitié des liaisons amide par molécule.

6. Peptide selon la revendication 3, comportant une des séquences suivantes :
F- (N-Me) GA- (N-Me) IL, NF- (N-Me) GA- (N-Me) IL, NNF- (N-Me) GA- (N-Me) IL, SNNF- (N-Me) GA- (N-Me) IL, NF- (N-Me) GA- (N-Me) ILSS, SNNF-(N-Me) GA- (N-Me) IL, FG- (N-Me) AI- (N-Me) L, NFG- (N-Me) AI- (N-Me) L, NNFG- (N-Me) AI- (N-Me) L, SNNFG- (N-Me) AI- (N-Me) L, NFG- (N-Me) AI- (N-Me) LSS, SNNFG- (N-Me) AI- (N-Me) LSS, FGA- (N-Me) IL, NFGA-(N-Me) IL, NNFGA- (N-Me) IL, SNNFGA- (N-Me) IL, NFGA- (N-Me) ILSS, SN- (N-Me) NFGAILSS, (N-Me) SN- (N-Me) NFGAILSS, (N-Me) SN- (N-Me) NF- (N-Me) GAILSS, K- (N-Me) GA- (N-Me) II, NK- (N-Me) GA- (N-Me) II, SNK- (N-Me) GA- (N-Me) II, GSNK- (N-Me) GA- (N-Me) II, NK- (N-Me) GA-(N-Me) IIGL, GSNK- (N-Me) GA- (N-Me) II, KG- (N-Me) AI- (N(Me) I, NKG-(N-Me) AI- (N-Me) I, SNKG- (N-Me) AI- (N-Me) I, GSNKG- (N-Me) AI- (N-Me) I, NKG- (N-Me) AI- (N-Me) IGL, GSNKG- (N-Me) AI- (N-Me) IGL, KGA-(N-Me) II, NKGA- (N-Me) II, SNKGA- (N-Me) II, GSNKGA- (N-Me) IIGL, GS- (N-Me) NKGAIIGL, (N-Me) GS- (N-Me) NKGAIIGL, (N-Me) GS- (N-Me) NK- (N-Me) GAIIGL, A- (N-Me) GA- (N-Me) VV, AA- (N-Me) GA- (N-Me)VV, AAGA-(N-Me)VVGG, HV-(N-Me)AAGAVVGG,(N-Me)HV- (N-Me) AAGAVVGG, et (N-Me) HV- (N-Me) AA- (N-Me) GAVVGG.

7. Utilisation d'un peptide comportant jusqu'à 15 aminoacides qui est formé d'une des séquences peptidiques suivantes :
FGAIL, NFGAIL, NNFGAIL, SNNFGAIL, NFGAILSS ou SNNFGAILSS ou qui la renferme, en vue de la production d'un médicament en tant qu'inhibiteur de la formation de la substance amyloïde et/ou de la cytotoxicité de la protéine amyline (IAPP) pour le traitement et/ou le diagnostic du diabète sucré de type II.

8. Utilisation d'un peptide comportant jusqu'à 15 aminoacides qui est formé d'une des séquences peptidiques suivantes :
KGAII, NKGAII, SNKGAII, GSNKGAII, NKGAIIGL ou GSNKGAIIGL ou qui la contient en vue de la production d'un médicament en tant qu'inhibiteur de la formation de la substance amyloïde et/ou de la cytotoxicité de la protéine du peptide β-amyloïde (β-AP) pour le traitement et/ou pour le diagnostic de la maladie d'Alzheimer.

9. Utilisation d'un peptide comportant jusqu'à 15 aminoacides qui est formé d'une des séquences peptidiques suivantes :
AGAVV, AAGAVV, VAAGAVV, HVAAGAVV, AAGAVVGG ou HVAAGAVVGG ou qui la renferme, en vue de la production d'un médicament comme inhibiteur de la formation de la substance amyloïde et/ou de la cytotoxicité de la protéine de prion (PrP) pour le traitement et/ou pour le diagnostic de l'encéphalopatie spongiforme.

10. Utilisation d'un peptide selon l'une quelconque des revendications 3 à 6,
pour autant que celui-ci renferme la séquence GAIL, en vue de la production d'un médicament en tant qu'inhibiteur de la formation de substance amyloïde et/ou de la cytotoxicité de la protéine amyline (IAPP) pour le traitement et/ou le diagnostic du diabète sucré de type II, pour autant que celui-ci renferme la séquence GAII, en vue de la production d'un médicament en tant qu'inhibiteur de la formation de la substance amyloïde et/ou de la cytotoxicité de la protéine de peptide β-amyloïde (β-AP) pour le traitement et/ou le diagnostic de la maladie d'Alzheimer, et
pour autant que celui-ci renferme la séquence GAVV en vue de la production d'un médicament en tant qu'inhibiteur de la formation de substance amyloïde et/ou de la cytotoxicité de la protéine de prion (Pr-P) pour le traitement et/ou le diagnostic de l'encéphalopathie spongiforme.
